# EUROPÄISCHE PATENTSCHRIFT

(11) **EP 1 565 235 B1**
(45) Veröffentlichungstag und Bekanntmachung des Hinweises auf die Patenterteilung: **11.01.2012**
(21) Anmeldenummer: 03767565.9
(22) Anmeldetag: 17.11.2003
(51) Int. Cl.: A61N 1/39

(54) **VERFAHREN ZUM ÜBERPRÜFEN DER BETRIEBSFÄHIGKEIT EINES EXTERN ANZUWENDENDEN DEFIBRILLATORS**
METHOD FOR VERIFYING THE OPERABILITY OF A DEFIBRILLATOR THAT IS TO BE USED EXTERNALLY
PROCEDE POUR VERIFIER LA FONCTIONNALITE D'UN DEFIBRILLATEUR A USAGE EXTERNE

(30) Priorität: 19.11.2002 DE 10254480
(43) Veröffentlichungstag der Anmeldung: 24.08.2005
(73) Patentinhaber: Metrax GmbH, D-78628 Rottweil (DE)
(72) Erfinder: BUCHER, Heinz, 78615 Rottweil (DE)
(74) Vertreter: Fleck, Hermann-Josef
(86) Internationale Anmeldenummer: PCT/EP2003/012856
(87) Internationale Veröffentlichungsnummer: WO 2004/045713

(56) Entgegenhaltungen:
- EP-A- 0 671 687
- US-A- 5 097 830
- US-A- 5 879 374

## Beschreibung

Die Erfindung bezieht sich auf ein Verfahren zum Überprüfen der Betriebsfähigkeit eines extern anzuwendenden Defibrillators, bei dem aus einer Ruhebetriebsart heraus unterschiedliche Testabläufe ausgelöst und durchgeführt werden, die einen eine Programmspeichereinrichtung, eine Arbeitsspeichereinrichtung und ein Betriebssystem überprüfenden Start-Test und einen an diesen anschließenden, wesentliche Defibrillationskomponenten überprüfenden Kurz-Test umfassen.

Ein Verfahren dieser Art zum Überprüfen der Betriebsfähigkeit eines extern anzuwendenden Defibrillators ist in der EP-A 0 671 687 angegeben. Bei diesem bekannten Verfahren werden aus einer Ruhebetriebsart heraus automatisch verschiedene Testabläufe ausgelöst, die Prozessorkomponenten und auch Defibrillationskomponenten umfassen. Ein ähnliches Verfahren ist auch in der US-A 5,097,830 gezeigt. Auch hierbei folgen bestimmte Tests aufeinander.

Hierbei werden auch Laufzeit-Tests kontinuierlich während der Laufzeit des Defibrillators durchgeführt, in denen die Sicherheit und Wirksamkeit von Teilen des Defibrillators überprüft werden.

Verfahren zum Überprüfen der Betriebsfähigkeit eines extern anzuwendenden Defibrillators sind an sich bei herkömmlichen Defibrillatoren üblich, wobei ein Testablauf beispielsweise von einer Bedienperson oder Prüfpersonal auslösbar ist. Auch aus der WO 93/16759 ist ein Testverfahren für einen Defibrillator bekannt, wobei beispielsweise überprüft werden kann, ob von einem Hochspannungsteil gebildete Impulse für die Defibrillation geeignet sind. Der Hochspannungsteil besitzt dabei eine Entladeschaltung mit einer Energiequelle in Form eines Kondensators zum Speichern der Energie für den Defibrillationsimpuls. Zum Überprüfen werden Parameter erfasst, die aussagekräftig für den Defibrillationsimpuls sind, und werden mit vorbestimmten Werten verglichen. Das Prüfungsergebnis wird angezeigt. Auch ist bereits in dem zu der WO 93/16759 genannten Stand der Technik "Medical and Biological Engineering an Computing; November 1979; Sum, Dewhurst, Digital Cardiac Defibrillator Tester, 710-714, ein Defibrillator mit Testsystem angegeben.

Ein weiteres Testsystem für Defibrillatoren ist in der WO 94/27674 angegeben, wobei eine periodische Funktionsüberprüfung, Kalibrierung und Sicherheitsüberprüfung in dem Defibrillator durchgeführt werden. Die Überprüfung kann auch durch bestimmte Ereignisse ausgelöst werden, wie z.B. Vibration, Feuchtigkeit oder Temperatur. Ein ähnliches Testsystem bei einem Defibrillator ist auch in der US 5,879,374 angegeben.

Der Erfindung liegt die Aufgabe zugrunde, ein Verfahren der eingangs genannten Art mit mehreren verschiedenen Tests bereitzustellen, bei dem eine verbesserte Organisation von Abläufen verschiedener Tests bei erhöhter Zuverlässigkeit der Funktion erreicht wird.

Diese Aufgabe wird mit den Maßnahmen des Anspruches 1 gelöst. Hiernach ist vorgesehen, dass nach Durchführen des Start-Tests stets automatisch ein unterlagerter, selbstständig ablaufender Laufzeit-Test durchgeführt wird, bis in die Ruhebetriebsart zurückgegangen wird, wobei die Durchführung des Laufzeit-Tests in vorgegebenen oder vorgebbaren Zeitabständen zyklisch wiederholt wird und irgendwelche kritischen Fehler entsprechend vorgegebener oder vorgebbarer Fehlerkriterien festgestellt werden.

Mit diesen Maßnahmen wird erreicht, dass verschiedene Testabläufe organisiert in vorgegebener oder vorgebbarer Weise ablaufen, wobei auch unterschiedliche Kriterien in einem Programm zugrunde gelegt werden können und durch den nach Durchführen des Start-Tests stets automatisch durchgeführten Laufzeit-Test eine erhöhte Zuverlässigkeit der Funktion gegeben ist.

Eine vorteilhafte Ausgestaltung des Verfahrens besteht darin, dass die Testabläufe einen Langzeit-Test umfassen, der eine Überprüfung des Zustandes einer Energiequelle für den Defibrillationsimpuls und/oder einen Relais-Test zum Ankoppeln eines Relais von Patientenelektroden umfasst, und dass der Langzeit-Test nach vorgegebenen Zeitdauern automatisch und/oder nach Abruf durch einen Benutzer ausgelöst wird. Mit diesen Maßnahmen werden weitere Überprüfungsmöglichkeiten geboten, die jedoch weniger häufig, z.B. monatlich, durchgeführt werden. Dabei kann auch vorgesehen sein, dass ein derartiger erweiterter Test auf Anforderung zwischendurch ausgelöst werden kann.

Eine weitere vorteilhafte Ausgestaltung des Verfahrens wird dadurch erreicht, dass die Testabläufe einen benutzerassistierten Test umfassen, der bei Bedarf von einem Benutzer ausgelöst wird. Hierbei bestehen für einen Benutzer, insbesondere auch technisches Prüfpersonal, erweiterte Testmöglichkeiten, um z.B. bestimmte Funktionen gezielt überprüfen zu können.

Hierbei kann vorteilhaft vorgesehen sein, dass bei Auslösen des benutzer-assistierten Tests zuvor der Start-Test, der Kurz-Test, gegebenenfalls der Langzeit-Test und gegebenenfalls ein Voll-Test durchgeführt werden.

Für weitere Prüfungsmöglichkeiten können die Maßnahmen vorteilhaft sein, dass nach dem Kurz-Test oder dem Langzeit-Test ein Voll-Test folgt, bei dem eine Überprüfung des Zustandes einer Energiequelle für den Defibrillationsimpuls und/oder ein Relais-Test zum Ankoppeln eines Relais von Patientenelektroden erfolgt.

Für die Information eines Benutzers bzw. Überwachung oder Überprüfung durch Fachpersonal sind weiterhin die Maßnahmen von Vorteil, dass im Anschluss an den Kurz-Test, an gegebenenfalls den Langzeit-Test, an gegebenenfalls den Voll-Test und an gegebenenfalls den benutzer-assistierten Test eine jeweilige Fehlerabfrage durchgeführt wird und dass bei Feststellen eines Fehlers dieser gespeichert und/oder angezeigt wird. Mit dem Abspeichern der Fehler ergibt sich eine Protokollier- und Diagnosemöglichkeit. Auch kann eine Diagnose über eine Diagnoseschnittstelle drahtgebunden oder drahtlos erfolgen.

Hierbei besteht eine vorteilhafte Ausbildung darin, dass bei dem Laufzeit-Test eine Überprüfung der Software, eine unberechtigte Inbetriebnahme, interne elektrische Spannungen und/oder ein Batteriezustand überprüft wird/werden.

Die Erfindung wird nachfolgend anhand von Ausführungsbeispielen unter Bezugnahme auf die Zeichnungen näher erläutert. Es zeigen:
- Fig.: 1 ein schematisches Ablaufdiagramm zu einem übergeordneten Test-Konzept,
- Fig. 2: ein schematisches Ablaufdiagramm zu einem Laufzeit-Test,
- Fig. 3: ein schematisches Ablaufdiagramm zu einem Start-Test,
- Fig. 4: ein schematisches Ablaufdiagramm zu einem Kurz-Test,
- Fig. 5: ein schematisches Ablaufdiagramm zu einem Langzeit-Test,
- Fig. 6: ein schematisches Ablaufdiagramm zu einem Voll-Test und
- Fig. 7: ein schematisches Ablaufdiagramm zu einem benutzer-assistierten Test.

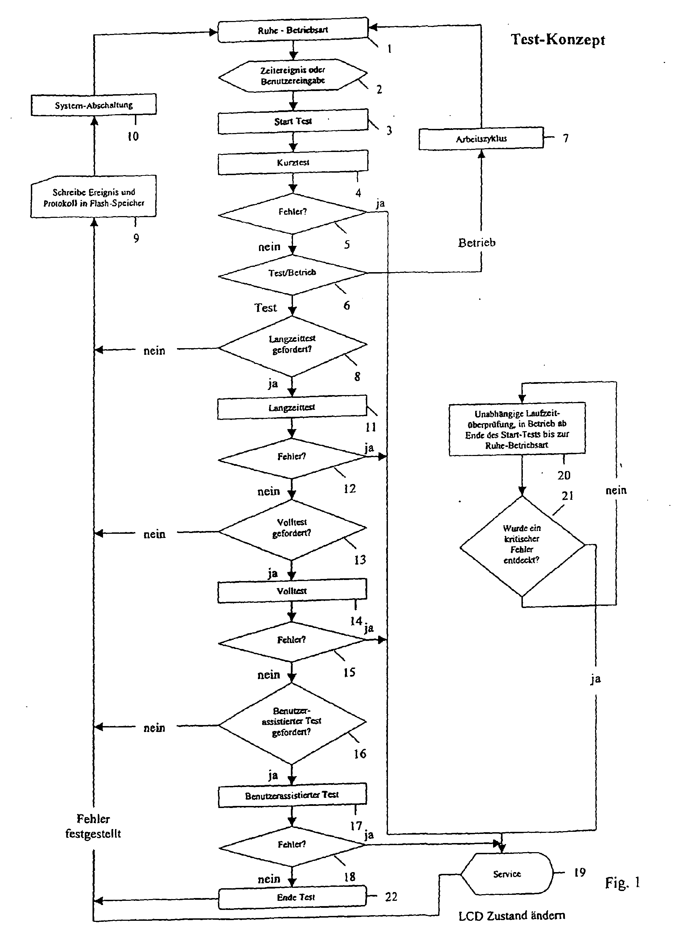

Das bei dem Defibrillator, insbesondere einem automatischen externen Defibrillator (AED), vorgesehene Testsystem beinhaltet ein beispielsweise in einem Mikrorechner oder Mikrocontroller oder einer vergleichbaren logischen Schaltung hinterlegtes Testprogramm mit einem übergeordneten Test-Konzept, mit dem verschiedene, modulartig aufgebaute und aufrufbare Einzeltests in vorgegebener oder vorgebbarer Weise organisiert werden. Fig. 1 zeigt ein Ausführungsbeispiel für ein Test-Konzept.

Aus einer Ruhe-Betriebsart (Schritt 1) heraus erfolgt z.B. nach einer vorgegebenen oder vorgebbaren Zeit oder auf eine Benutzereingabe hin (Schritt 2) ein Start-Test (Schritt 3) und im Anschluss daran ein Kurz-Test (Schritt 4). Dann wird überprüft, ob ein Fehler vorliegt (Schritt 5). Liegt ein Fehler vor, wird eine entsprechende Anzeige (optisch und/oder akustisch) erzeugt (Schritt 19). Liegt kein Fehler vor, wird anschließend überprüft, ob ein Testablauf oder ein Betriebsablauf vorliegt (Schritt 6), der über eine Benutzereingabe gewählt sein kann. Liegt ein Betriebsablauf (Arbeitszyklus, Schritt 7) vor, wird wieder in die Ruhe-Betriebsart (Schritt 1) übergegangen. Liegt ein Testablauf vor, wird überprüft, ob ein Langzeit-Test gefordert ist (Schritt 8) und dieser gegebenenfalls durchgeführt (Schritt 11). Ist kein Langzeit-Test gefordert, wird das Ereignis aufgeschrieben und in z.B. einem Flash-Speicher protokolliert (Schritt 9). Dann wird das System abgeschaltet (Schritt 10) und wieder in die Ruhebetriebsart (Schritt 1) übergegangen. Ist ein Langzeit-Test gefordert und wird ein Fehler festgestellt (Schritt 12), wird eine entsprechende Anzeige auf der LCD-Anzeige oder einer entsprechenden optischen Anzeige erzeugt (Schritt 19) und/oder akustisch dargestellt. Liegt kein Fehler vor, wird abgefragt, ob ein Voll-Test angefordert ist (Schritt 3), der z.B. nach einer entsprechenden Zeitdauer oder aufgrund einer manuellen Anforderung ausgelöst werden kann. Ist kein Voll-Test gefordert, wird das Ereignis aufgeschrieben und in z.B. dem Flash-Speicher protokolliert (Schritt 9). Dann wird das System abgeschaltet (Schritt 10) und wieder in die Ruhe-Betriebsart (Schritt 1) übergegangen. Ist ein Voll-Test gefordert, wird dieser durchgeführt (Schritt 14) und anschließend abgefragt, ob ein Fehler vorliegt (Schritt 15). Liegt ein Fehler vor, wird auf der Anzeige ein entsprechender Fehlerzustand wiedergegeben (Schritt 19). Liegt kein Fehler vor, wird überprüft, ob ein benutzer-assistierter Test durchgeführt werden soll (Schritt 16). Ist kein benutzer-assistierter Test gefordert, wird wieder über die Schritte 9 und 10 in die Ruhe-Betriebsart übergegangen (Schritt 1). Ist ein benutzer-assistierter Test angefordert, wird dieser durchgeführt (Schritt 17) und abgefragt, ob ein Fehler vorliegt (Schritt 18). Liegt ein Fehler vor, wird dieser zur Anzeige gebracht (Schritt 19). Liegt kein Fehler vor, wird zum Ende der Tests übergegangen (Schritt 22) und anschließend über die Schritte 9 und 10 in die Ruhe-Betriebsart (Schritt 1) übergegangen.

Falls ein Fehler festgestellt und angezeigt wird, wird wieder über die Schritte 9 und 10 in die Ruhe-Betriebsart (Schritt 1) zurückgekehrt, wobei der Fehlerzustand aber weiterhin angezeigt und/oder gespeichert bleiben kann, um eine entsprechende Auswertung durch den Benutzer und/oder durch Fachpersonal vorzunehmen.

Ab dem Ende des Start-Tests (Schritt 3) bis zu einer Rückkehr zur Ruhe-Betriebsart (Schritt 1) wird ein unabhängiger Laufzeit-Test (Schritt 20) durchgeführt und überprüft, ob dabei irgendein kritischer Fehler festgestellt wird (Schritt 21). Falls ein kritischer Fehler festgestellt wird, wird dieser zur Anzeige gebracht (Schritt 19). Wird kein kritischer Fehler festgestellt, wird der Laufzeit-Test (Schritt 20) wiederholt durchgeführt.

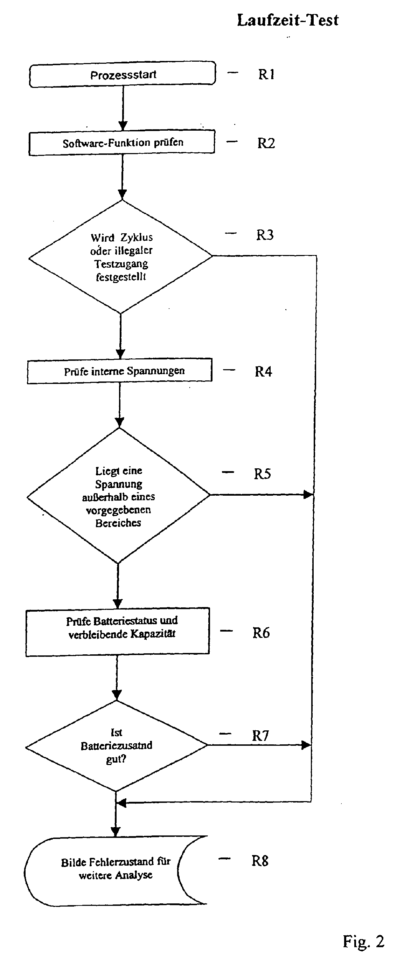

Ein Beispiel für einen Laufzeit-Test ist in Fig. 2 näher dargestellt. Nach einem Prozess-Start (Schritt R1) wird die Software-Funktion überprüft (Schritt R2). Wird dabei ein fehlerhafter Ablauf oder ein unberechtigter Zugang festgestellt (Schritt R3), wird ein Fehlerzustand für eine weitere Analyse gebildet (Schritt R8). Wird kein fehlerhafter Zyklus oder unberechtigter Zugang festgestellt, werden interne Spannungen überprüft (Schritt R4). Liegt eine Spannung über einer vorgegebenen oder vorgebbaren Grenze (Schritt R5) vor, wird ein Fehlerzustand für eine weitere Analyse gebildet (Schritt R8). Liegt keine Spannung außerhalb einer vorgegebenen Grenze oder eines vorgegebenen Bereiches, wird der Batteriezustand und eine verbleibende Kapazität überprüft (Schritt R6). Ist der Batteriezustand fehlerhaft, wird ein entsprechender Fehlerzustand für eine weitere Analyse gebildet, während bei Nichtvorliegen eines Fehlers auch eine entsprechende Anzeige erzeugt wird.

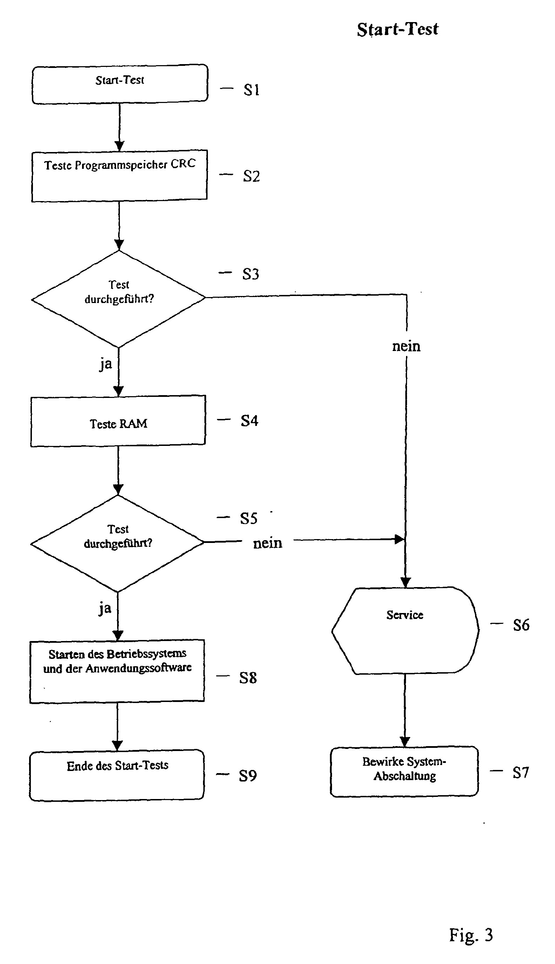

In Fig. 3 ist ein Ausführungsbeispiel für einen Start-Test näher dargestellt. Nach dem Auslösen des Start-Tests (Schritt S1) wird ein Programmspeicher getestet (Schritt S2). Läuft der Test nicht fehlerfrei ab (Schritt S3), wird ein entsprechender Fehler angezeigt (Schritt S6). Anschließend wird eine System-Abschaltung bewirkt (Schritt S7). Läuft die Programmprüfung fehlerfrei ab, wird der Arbeitsspeicher (RAM) überprüft (Schritt S4). Läuft die Überprüfung des Arbeitsspeichers nicht fehlerfrei ab (Schritt S5), erfolgt eine entsprechende Anzeige (Schritt S6) und eine System-Abschaltung wird bewirkt (Schritt S7). Tritt bei der Überprüfung des Arbeitsspeichers kein Fehler auf, werden das Betriebssystem und die Anwendungssoftware gestartet (Schritt S8) und anschließend wird der Start-Test beendet (Schritt S9).

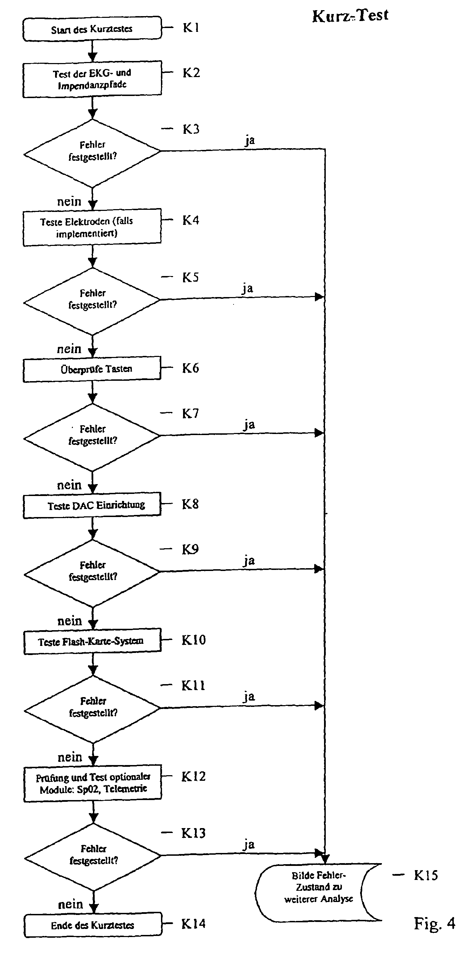

Fig. 4 zeigt ein Ausführungsbeispiel für einen Kurz-Test. Nach dem Start des Kurz-Tests (K1) erfolgt eine Überprüfung eines EKG-Kanals und von Impedanz-Kanälen (Schritt K2). Wird dabei ein Fehler festgestellt (Schritt K3), wird ein entsprechender Fehlerzustand zur weiteren Analyse angezeigt (Schritt K15). Wird kein Fehler erkannt, wird eine Elektrodenüberprüfung durchgeführt, falls eine derartige Überprüfungsmöglichkeit bzw. überprüfbare Elektroden vorhanden sind (Schritt K4). Wird dabei ein Fehler festgestellt (Schritt K5), wird ein Fehlerzustand zur weiteren Analyse gebildet (Schritt K15). Wird kein Fehler festgestellt, werden Taster, Schalter oder entsprechende Betätigungs- oder Auslöseglieder (z.B. Sprachsteuerung) überprüft (Schritt K6). Wird dabei ein Fehler festgestellt (Schritt K7), wird ein Fehlerzustand zur weiteren Analyse erzeugt (Schritt K15). Wird kein Fehler festgestellt, wird eine Digital-Analog-Wandler-Einrichtung überprüft (Schritt K8). Wird dabei ein Fehler festgestellt, wird ein Fehlerzustand zur weiteren Analyse erzeugt (Schritt K15). Wird kein Fehler festgestellt, wird zur Überprüfung einer Flash-Speichervorrichtung übergegangen (Schritt K10). Wird dabei ein Fehler festgestellt, wird ein entsprechender Fehlerzustand zur weiteren Analyse erzeugt (Schritt K15). Wird kein Fehler festgestellt, werden optional vorhandene Module überprüft, wie z.B. Sp02, Telemetrie (Schritt K12). Wird dabei ein Fehler festgestellt, wird ein entsprechender Fehlerzustand zur weiteren Analyse erzeugt (Schritt K15). Wird kein Fehler festgestellt, wird zum Ende des Kurz-Tests übergegangen (Schritt K14). Auch nach Erzeugen eines Fehlerzustandes zur weiteren Analyse wird nach Anzeige desselben und/oder Abspeicherung desselben zum Ende des Kurz-Tests übergegangen (Schritt K14).

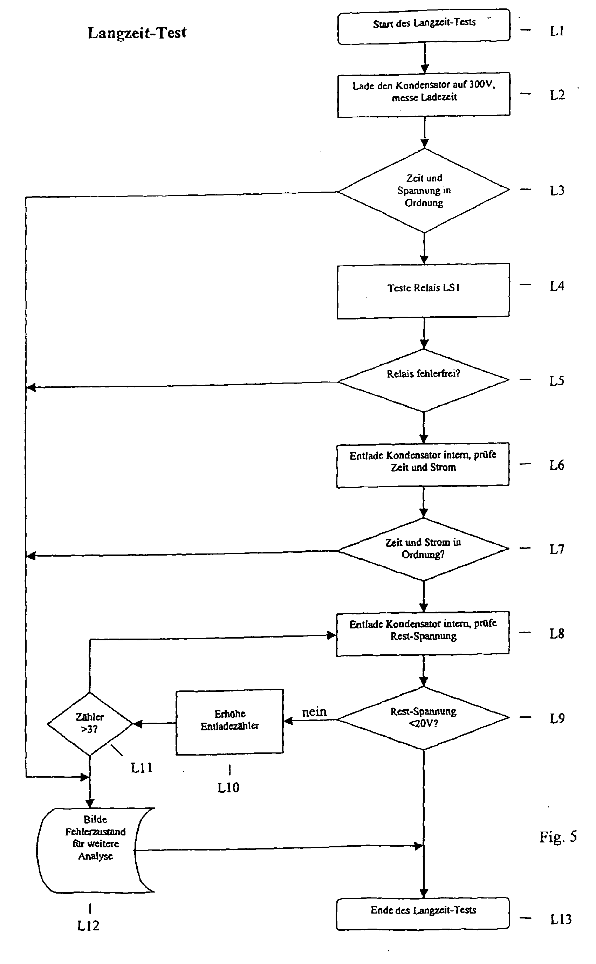

Fig. 5 zeigt ein Beispiel für einen Langzeit-Test. Nach dem Start des Langzeit-Tests (Schritt L1) wird ein Energiespeicher, insbesondere ein Kondensator oder eine Kondensatoranordnung auf eine Spannung zwischen z.B. 100 V und 600V, beispielsweise zwischen 300 V und 500 V aufgeladen und die Ladezeit bis zum Erreichen der vorgegebenen Spannung gemessen (Schritt L2). Entsprechen die Ladezeit und die Spannung nicht den Vorgaben (Schritt L3), wird ein entsprechender Fehlerzustand zur weiteren Analyse erzeugt (Schritt L12). Entsprechen die Zeit und die Spannung den Vorgaben, wird ein Koppel-Relais LS1 geprüft (Schritt L4). Wird ein Fehler des Relais festgestellt (Schritt L5), wird zur Fehleranzeige und weiteren Analyse übergegangen (Schritt L12). Liegt kein Relaisfehler vor, wird der Energiespeicher bzw. Kondensator intern entladen und die Zeit und der Strom oder die Spannung werden überprüft (Schritt L6). Entsprechen die Zeit und der Strom bzw. die Spannung nicht den Vorgaben, wird ein Fehlerzustand zur weiteren Analyse erzeugt (Schritt L12). Entsprechen die Zeit und der Strom bzw. die Spannung den Vorgaben, wird die Energiequelle bzw. der Kondensator intern entladen und eine Rest-Spannung überprüft. Beträgt die Restspannung nicht weniger als eine vorgegebene Spannung, von z.B. in der Größenordnung von 10 V oder einigen 10 V, beispielsweise < 20 V (Schritt L9), wird der Zustand eines Entladezähler inkrementiert (Schritt L10). Wird eine Zählgrenze, z.B. der Wert 3 überschritten (Schritt L11), wird ein Fehlerzustand zur weiteren Analyse gebildet (Schritt L12). Ist die Zählgrenze nicht überschritten, wird zu dem Schritt L8 zurückgekehrt und der Kondensator intern entladen und die Rest-Spannung überprüft. Beträgt die RestSpannung weniger als die vorgegebene Spannung von z.B. 20 V, wird der Langzeit-Test beendet (Schritt L13). Andernfalls werden die Schritte L10, L11 erneut durchlaufen. Der Langzeit-Test wird auch beendet, nachdem ein Fehlerzustand für die weitere Analyse gebildet und angezeigt und/oder gespeichert ist.

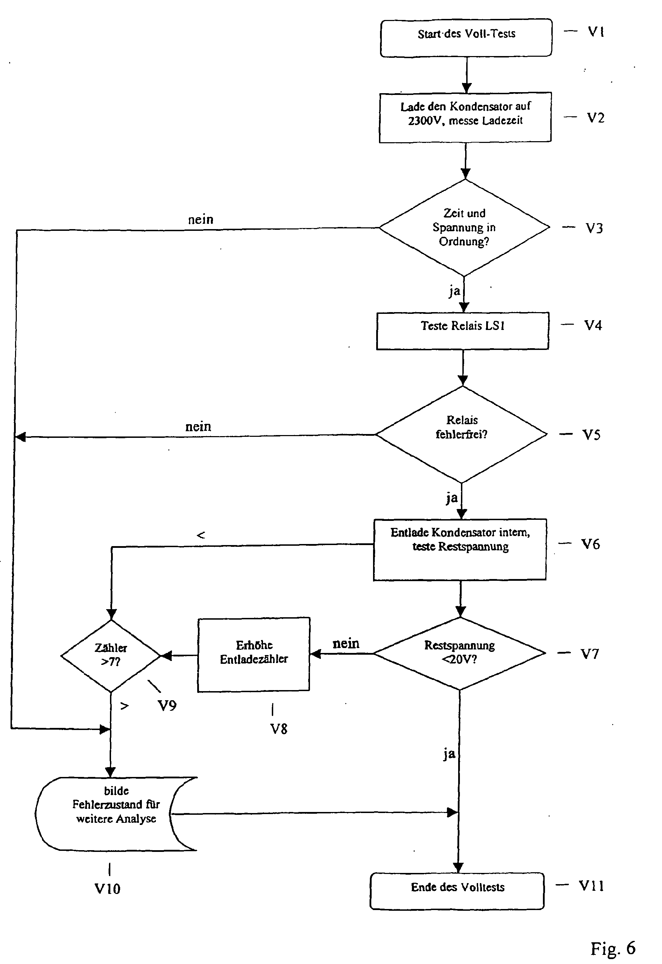

Fig. 6 zeigt ein Ausführungsbeispiel für einen Voll-Test. Nach dem Start des Voll-Tests (V1) wird eine Energiequelle, insbesondere ein Kondensator oder eine Kondensatoranordnung auf z.B. eine Spannung in der Größenordnung eines oder einiger kV, beispielhaft 2.300 V aufgeladen und die Ladezeit gemessen (Schritt V2). Entsprechen die Ladezeit und die Spannung nicht den Vorgaben, wird ein Fehlerstatus zur weiteren Analyse gebildet (Schritt V10). Entsprechen Zeit und Spannung den Vorgaben, wird ein Koppel-Relais zum Umschalten zwischen einem Test-Widerstand und Patienten-Elektroden oder lediglich zum Anschalten von PatientenElektroden überprüft (Schritt V4). Ist das Relais LS1 fehlerhaft (Schritt V5), wird ein entsprechender Fehlerzustand für eine weitere Analyse gebildet (Schritt V10). Ist das Relais LS1 fehlerfrei, wird die Energiequelle, insbesondere der Kondensator oder die Kondensatoranordnung intern entladen und die Rest-Spannung überprüft. Unterschreitet die Rest-Spannung einen vorgegebenen Wert von z.B. etwa 10V oder einigen 10 V, z.B. 20 V nicht (Schritt V7), so wird ein Entladezähler inkrementiert. Überschreitet der Entladezähler einen vorgegebenen Zählwert, wird ein Fehlerzustand zur weiteren Analyse gebildet (Schritt V10). Ist der Zählwert nicht überschritten, wird der Kondensator weiter intern entladen und die Rest-Spannung überprüft (Schritt V6). Unterschreitet die Rest-Spannung den Vorgabewert von z.B. 20 V, wird der Voll-Test beendet (Schritt V11). Andernfalls werden die Testschritte V8,V9 erneut durchlaufen. Der Voll-Test wird auch beendet (Schritt V11), wenn ein Fehler-zustand gebildet (Schritt V10) ist und angezeigt und/oder gespeichert wird.

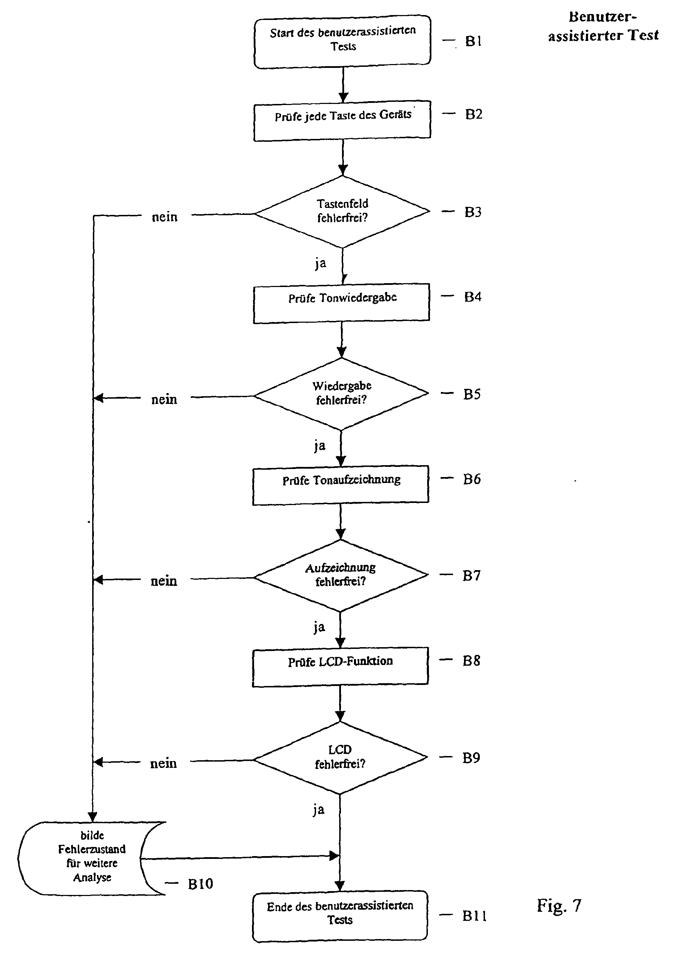

Fig. 7 zeigt ein Ausführungsbeispiel für einen benutzer-assistierten Test. Nach dem Start des benutzer-assistierten Tests (Schritt B1) werden Taster, Schalter oder entsprechende Eingabeelemente, wie z.B. auch Spracheingabeelemente überprüft (Schritt B2). Sind die Eingabeelemente fehlerhaft (Schritt B3), wird ein entsprechender Fehlerzustand für die weitere Analyse gebildet (Schritt B10). Sind die Eingabeelemente, wie etwa ein Tastenfeld fehlerfrei, wird eine evtl. Ton-Wiedergabe überprüft (Schritt B4). Ist die Ton-Wiedergabe fehlerhaft (Schritt B5), wird ein Fehlerstatus zur weiteren Analyse gebildet (Schritt B10). Ist die Ton-Wiedergabe fehlerfrei, wird eine evtl. vorhandene Ton-Aufzeichnung überprüft (Schritt B6). Ist die Aufzeichnung fehlerhaft (Schritt B7), wird ein Fehlerzustand für die weitere Analyse gebildet (Schritt B10). Ist die Ton-Aufzeichnung fehlerfrei, wird die Anzeige-Funktion, beispielsweise eine LCD-Anzeige überprüft (Schritt B8). Ist die Anzeige fehlerhaft (Schritt B9), wird ein entsprechender Fehlerzustand für die weitere Analyse gebildet (Schritt B10). Ist die Anzeige fehlerfrei, kann der benutzer-assistierte Test erneut gestartet werden oder er wird beendet (Schritt B11). Schritt B11 erfolgt auch, nachdem ein Fehlerzustand für die weitere Analyse gebildet und eine entsprechende Anzeige erzeugt oder der Fehlerzustand gespeichert ist.

Mit dem beschriebenen Test-Konzept können die modulartig aufgebauten und eingebundenen Einzeltests in gewünschter vorgegebener Reihenfolge geeignet durchlaufen werden, wobei durch eine Programmierung die Testfolge, ihre jeweilige Häufigkeit und ihr Auslösen auch in Abhängigkeit voneinander sowie in Abhängigkeit festgestellter Fehler vorgegegen werden können. Auch ist es möglich, einzelne Tests wegzulassen oder unter bestimmten Umständen in Abhängigkeit vorgegebener oder vorgebbarer Kriterien zu überspringen. Auch können einzelne Schritte der Einzeltests übersprungen oder andere Testschritte mittels des Programms eingefügt werden.

## Patentansprüche

1. Verfahren zum Überprüfen der Betriebsfähigkeit eines extern anzuwendenden Defibrillators, bei dem aus einer Ruhebetriebsart (1) heraus nach einer vorgegebenen oder vorgebbaren Zeit oder auf eine Benutzereingabe (2) unterschiedliche Testabläufe ausgelöst und durchgeführt werden, die einen eine Programmspeichereinrichtung (S2), eine Arbeitsspeichereinrichtung und ein Betriebssystem (S8) überprüfenden Start-Test (3) und einen an diesen anschließenden, wesentliche Defibrillationskomponenten überprüfenden Kurz-Test (4) umfassen,
**dadurch gekennzeichnet,**
**dass** nach Durchführen des Start-Tests (3) stets automatisch ein unterlagerter, selbstständig ablaufender Laufzeit-Test (20) durchgeführt wird, bis in die Ruhebetriebsart (1) zurückgegangen wird, wobei die Durchführung des Laufzeit-Tests (20) in vorgegebenen oder vorgebbaren Zeitabständen zyklisch wiederholt wird, bei dem Laufzeit-Test (20) eine Überprüfung (R2) der Software, eine unberechtigte Inbetriebnahme (R3), interne elektrische Spannungen (R4) und/oder ein Batteriezustand überprüft wird/werden und irgendwelche kritischen Fehler entsprechend vorgegebener oder vorgebbarer Fehlerkriterien festgestellt werden,
**dass** nach Durchführen des Kurz-Tests (4) eine Fehlerabfrage (5) vorgenommen wird und, wenn kein Fehler vorliegt, abgefragt wird, ob ein Testablauf oder ein Betriebsablauf zum Durchführen eines Arbeitszyklus (7) des Defibrillators vorliegt (6), und
**dass** dann, wenn ein Betriebsablauf gewählt wurde, nach dem Arbeitszyklus (7) wieder in die Ruhebetriebsart (1) übergegangen wird.

2. Verfahren nach Anspruch 1,
**dadurch gekennzeichnet,**
**dass** die Testabläufe des Weiteren nach der Abfrage (6), ob ein Testablauf oder ein Betriebsablauf vorliegt, einen Langzeit-Test (13) umfassen, der eine Überprüfung des Zustandes einer Energiequelle für den Defibrillationsimpuls und/oder
einen Relais-Test (L4) zum Ankoppeln eines Relais von Patientenelektroden umfasst, und
**dass** der Langzeit-Test (13) nach vorgegebenen Zeitdauern automatisch und/oder nach Abruf durch einen Benutzer ausgelöst wird.

3. Verfahren nach Anspruch 1 oder 2,
**dadurch gekennzeichnet,**
**dass** die Testabläufe einen benutzer-assistierten Test (17) umfassen, der bei Bedarf von einem Benutzer ausgelöst wird und bei dem Taster, Schalter oder Spracheingabeelemente, eine Tonwiedergabe oder eine Anzeige-Funktion überprüft werden.

4. Verfahren nach Anspruch 3,
**dadurch gekennzeichnet,**
**dass** bei Auslösen des benutzer-assistierten Tests (17) zuvor der Start-Test (3), der Kurz-Test (4), der Langzeit-Test (11) und ein gegebenenfalls vorhandener Voll-Test (14) durchgeführt werden.

5. Verfahren nach einem der vorhergehenden Ansprüche,
**dadurch gekennzeichnet,**
**dass** nach dem Kurz-Test (4) oder dem Langzeit-Test (11) ein Voll-Test (14) folgt, bei dem eine Überprüfung (V2) des Zustandes einer Energiequelle für den Defibrillationsimpuls und/oder ein Relais-Test (V5) zum Ankoppeln eines Relais von Patientenelektroden erfolgt.

6. Verfahren nach einem der vorhergehenden Ansprüche,
**dadurch gekennzeichnet,**
**dass** im Anschluss an den Kurz-Test (4), an gegebenenfalls den Langzeit-Test (11), an gegebenenfalls den Voll-Test (14) und an gegebenenfalls den benutzer-assistierten Test (17) eine jeweilige Fehlerabfrage durchgeführt wird und dass bei Feststellen eines Fehlers dieser gespeichert und/oder angezeigt wird.

## Claims

1. A method for testing the operational capability of a defibrillator to be applied externally, with which, starting from a resting operating mode (1), after a specified or specifiable time or in response to a user input (2), various test procedures, which comprise a start test (3) testing a program storage device (S2), a random access memory device and an operating system (S8); and a short test (4) following the latter and testing substantial defibrillation components, are triggered and implemented,
**characterised in that**,
after the implementation of the start test (3), a subordinate, automatically running delay time test (20) is always implemented automatically until the resting operating mode (1) is restored, wherein the implementation of the delay time test (20) is repeated in a cyclical manner at specified or specifiable time intervals;
in the delay time test (20), a testing (R2) of the software, an unauthorised start-up (R3), internal electrical voltages (R4) and/or a battery status is/are tested, and any critical errors are determined according to specified or specifiable error criteria,
that after the implementation of the short test (4), an error interrogation (5) is implemented and, if no errors are present, an interrogation is implemented regarding whether a test procedure or an operating procedure for the implementation of an operational cycle (7) of the defibrillator is present (6), and
that if an operational procedure has been selected, the resting operating mode (1) is restored after the operational cycle (7).

2. The method according to claim 1,
**characterised in that**,
after the interrogation (6) regarding whether a test procedure or an operational procedure is present, the test procedures further comprise a long-term test (13), which comprises a testing of the status of an energy source for the defibrillation impulse and/or
a relay test (L4) for the coupling of a relay of patient electrodes, and
that the long-term test (13) is triggered automatically after specified intervals and/or on request by a user.

3. The method according to claim 1 or 2,
**characterised in that**
the test procedures comprise a user-assisted test (17), which is triggered as required by a user, and in which probes, switches or voice-activated input elements, a sound reproduction or display function are tested.

4. The method according to claim 3,
**characterised in that**,
when the user-assisted test (17) is triggered, the start test (3), the short test (4), the long-term test (11) and an optionally provided full test (14) are implemented first.

5. The method according to any one of the preceding claims,
**characterised in that**
a full test (14), in which a testing (V2) of the status of an energy source for the defibrillation impulse and/or a relay test (V5) for the coupling of a relay of patient electrodes is implemented, follows after the short test (4) or the long-term test (11).

6. The method according to any one of the preceding claims,
**characterised in that**,
following the short test (4), an error interrogation is implemented, in each case optionally on the long-term test (11), optionally on the full test (14) and optionally on the user-assisted test (17), and, if an error is determined, this is buffered and/or displayed.

## Revendications

1. Procédé pour vérifier la fonctionnalité d'un défibrillateur à usage externe, dans lequel, à partir d'un mode de repos (1), différentes séquences de test sont déclenchées et exécutées après un temps prédéfini ou prédéfinissable ou sur une entrée de l'utilisateur (2), qui comprennent un test initial (3) qui vérifie un dispositif mémoire de programme (S2), un dispositif mémoire de travail et un système d'exploitation, et un test court (4) faisant suite à celui-ci, qui vérifie des composants essentiels de défibrillation,
**caractérisé en ce**
**qu'**après exécution du test initial (3), un test de durée de marche (20) subordonné, se déroulant de manière autonome, est toujours exécuté automatiquement jusqu'au retour dans le mode de repos (1), l'exécution du test de durée de marche (20) étant répétée cycliquement à intervalles de temps prédéfinis ou prédéfinissables, lors duquel test de durée de marche (20) a lieu une vérification (R2) du logiciel, d'une mise en service non autorisée (R3), de tensions électriques internes (R4) et/ou d'un état de batterie et la constatation de défauts critiques quelconques selon des critères de défaut prédéfinis ou prédéfinissables,
**qu'**après exécution du test court (4) a lieu une interrogation de défauts (5) et, si aucun défaut n'est présent, une interrogation est effectuée pour savoir si une séquence de test ou une séquence de fonctionnement pour l'exécution d'un cycle de travail (7) du défibrillateur est présente (6), et
**que**, quand une séquence de fonctionnement a été choisie, un retour dans le mode de repos (1) a lieu après le cycle de travail (7).

2. Procédé selon la revendication 1,
**caractérisé en ce**
**que** les séquences de test comprennent de plus, après l'interrogation (6) pour savoir si une séquence de test ou une séquence de fonctionnement est présente, un test de longue durée (13) qui comprend une vérification de l'état d'une source d'énergie pour l'impulsion de défibrillation et/ou
un test de relais (L4) pour le couplage d'un relais d'électrodes de patient, et que le test de longue durée (13) est déclenché automatiquement après des durées prédéfinies et/ou sur appel par un utilisateur.

3. Procédé selon la revendication 1 ou 2,
**caractérisé en ce**
**que** les séquences de test comprennent un test assisté par l'utilisateur (17) qui est déclenché en cas de besoin par un utilisateur et au cours duquel des boutons, des commutateurs ou des éléments d'entrée vocale, une lecture sonore ou une fonction d'affichage sont vérifiés.

4. Procédé selon la revendication 3,
**caractérisé en ce**
**qu'**en cas de déclenchement du test assisté par l'utilisateur (17), le test initial (3), le test court (4), le test de longue durée (11) et un test complet (14) éventuellement présent sont exécutés au préalable.

5. Procédé selon une des revendications précédentes,
**caractérisé en ce**
**qu'**après le test court (4) ou le test de longue durée (11) est exécuté un test complet (14) au cours duquel a lieu une vérification (V2) de l'état d'une source d'énergie pour l'impulsion de défibrillation et/ou un test de relais (V5) pour le couplage d'un relais d'électrodes de patient.

6. Procédé selon une des revendications précédentes,
**caractérisé en ce**
**qu'**à la suite du test court (4), une interrogation de défaut est effectuée chaque fois lors du test de longue durée (11) éventuel, lors du test complet (14) éventuel et lors du test assisté par l'utilisateur (17) éventuel et que si un défaut est constaté, celui-ci est enregistré et/ou affiché.
